# EUROPÄISCHE PATENTANMELDUNG

(11) **EP 1 955 648 A1**
(43) Veröffentlichungstag der Anmeldung: **13.08.2008**
(21) Anmeldenummer: 07002500.2
(22) Anmeldetag: 06.02.2007
(51) Int. Cl.: A61B 5/00, A61B 19/02, G01D 7/12

(54) **Tasche für diagnostische Geräte**

(71) Anmelder: Roche Diagnostics GmbH, 68298 Mannheim (DE); F.HOFFMANN-LA ROCHE AG, 4070 Basel (CH)
(72) Erfinder: Kintzig, Hans, 67311 Tiefenthal (DE); Stadter, Ralf, 69234 Dielheim (DE); Kammer, Jutta, 69518 Abtsteinach (DE)

(57) **Zusammenfassung**

Es wird ein Modulares System zur akustischen Ausgabe von diagnostischen Messwerten beschrieben, das ein diagnostisches Messgerät mit einer Sendeeinheit für elektromagnetische Wellen enthält sowie ein Sprachausgabemodul mit einer Empfangseinheit für elektromagnetische Wellen und eine Tasche zur Aufbewahrung mindestens des Messgerätes und des Sprachausgabemoduls, mit einer ersten Halterung für das diagnostische Messgerät, die so ausgestaltet ist, dass das Gerät aus der Halterung herausnehmbar ist und bevorzugt nur in einer vorgegebenen Ausrichtung in der Halterung fixierbar ist und einer zweiten Halterung für das Sprachausgabemodul, die so ausgestaltet ist, dass das Gerät herausnehmbar ist und bevorzugt nur in einer vorgegebenen Ausrichtung in der Halterung fixierbar ist, dadurch gekennzeichnet, dass die erste Halterung und die zweite Halterung so innerhalb der Tasche zu einander angeordnet sind, dass bei Einlegen der Geräte in die erste und die zweite Halterung ein drahtloser Datenaustausch durch die räumliche Ausrichtung des Senders der Sendeeinheit und des Empfängers der Empfangseinheit zwischen den beiden Geräten stattfinden kann.

## Beschreibung

### Gebiet der Erfindung:

Die Erfindung betrifft eine Vorrichtung zum Transport von medizinischen Kleingeräten. Vorrichtungen zum Transport von medizinischen Geräten und deren Utensilien finden vielfältige Anwendung. Diese Vorrichtungen dienen zum einfachen Transport der Kleingeräte, so dass der Benutzer dieser Kleingeräte, diese einfach und gemeinsam transportieren kann und dabei die Geräte vor Stößen oder sonstigen Beeinflussungen während des Transportes geschützt sind. Besonders für Patienten, die Kleingeräte und dazugehörige Utensilien zur ständigen Selbstkontrolle von beispielsweise Blut- oder Urinproben mit sich führen müssen, ist es sehr hilfreich die Geräte und Utensilien in einer Vorrichtung zum Transport zusammen geschützt transportieren zu können.

### Stand der Technik

Im diagnostischen Bereich, vor allem im Selbstdiagnosebereich von z.B. Diabetikern benötigt der Patient zur Selbstdiagnose mindestens ein Messgerät und darüber hinaus verschiedene Utensilien wie beispielsweise Stechhilfen, Teststreifen und Lanzetten. Vorrichtungen zum Transport solcher Utensilien, beispielsweise in Form einer Tasche mit verschiedenen Halterungen für die unterschiedlichen Gegenstände sind im Stand der Technik hinlänglich bekannt. Bestehen die zu transportierenden Utensilien nicht nur aus einem Messgerät sondern aus verschiedenen Geräten die zeitweise einen Kommunikationsaustausch haben können, kann es notwendig sein die verschiedenen Geräte zur Datenübertragung miteinander zu verbinden.

Eine Möglichkeit dies innerhalb einer Tasche zu gewährleisten ist in dem Patent US 6,781,522 beschrieben. Hier wird in einem tragbaren Vorratsbehältnis sowohl ein medizinisches Messgerät als auch ein tragbares Rechnergerät bevorratet. Diese beiden Geräte können mit Hilfe einer integrierten Steckverbindung so miteinander verbunden werden, dass ein Datenaustausch ermöglicht wird. Nachteil dieser integrierten Steckverbindung zum Datenaustausch ist, dass sich das eingesteckte Gerät beim Transport lösen könnte und die integrierte Steckverbindung sich bei häufiger Benutzung abnutzt und gewartet werden muss. Außerdem sind in der Vorrichtung Kabel notwendig, die die Steckverbindungen miteinander in elektrischen Kontakt bringen. Diese Kabel können bei häufigem Gebrauch und häufigem Knicken unbrauchbar werden, so dass ein Wechsel notwendig ist. Solche elektrischen Steckverbindungen sind zusätzlich empfindlich gegenüber Feuchtigkeit, was bei Benutzung von beispielsweise Diabetikern, die häufig mit flüssigen Proben und Referenzmaterialien agieren müssen, zu Verschmutzungen der elektrischen Steckverbindung führen kann, da nicht auszuschließen ist, dass Flüssigkeiten verschüttet werden und sich in der Tasche und damit auch in den Steckverbindungen ausbreitet.

In der Patentanmeldung EP 1589334 wird die Bevorratung eines Messgerätes zusammen mit einem Vorratsbehälter für Testelemente beschrieben, wobei in die Halterungen für das Gerät und den Behälter eine Einheit integriert ist, um einen Informationsaustausch zwischen den beiden Geräten zu gewährleisten. Dies kann sowohl durch elektrische Verbindungen als auch durch das Aussenden bzw. Empfangen von optischen Signalen durchgeführt werden. Nachteil der in dieser Anmeldung beschriebenen Vorratstasche ist, dass elektronische oder optische Sende- bzw. Empfangseinheiten in die Tasche integriert werden müssen, um einen Informationsaustausch zwischen Gerät und Vorratsbehälter für Testelemente zu gewährleisten. Diese Integration von elektronischen Elementen in die Vorratstasche birgt zum einen die Gefahr, dass diese zusätzlichen elektronischen Elemente durch Fehlbenutzung beschädigt werden oder anderweitig unbrauchbar werden oder einen zusätzlichen Wartungsaufwand bedeuten. Auch hier können Flüssigkeiten, die bei der Benutzung des Gerätes in Kontakt mit den elektronischen Elementen gelangen, die elektrischen Elemente unbrauchbar machen und damit den Informationsaustausch zwischen den Geräten unmöglich machen. Des Weiteren bedeutet die Ankoppelung von Messgerät bzw. Vorratsbehälter an diese elektronischen Einheiten eine präzise Handhabung durch den Benutzer und somit eine Gefahr der Fehlbenutzung bzw. Zerstörung der elektronischen Teile durch den Benutzer.

Aus den Nachteilen des Standes der Technik ergibt sich die Aufgabe, ein tragbares Bevorratungsbehältnis für diagnostische Geräte und Utensilien zu entwickeln, dass eine einfachere und weniger störanfällige Kommunikation zwischen Messgeräten innerhalb des Vorratsbehältnisses gewährleistet.

### Allgemeine Beschreibung

Erfindungsgemäß wird ein modulares System zur akustischen Ausgabe von diagnostischen Messwerten beschrieben, enthaltend ein diagnostisches Messgerät mit einer Sendereinheit für elektromagnetische Wellen, ein Sprachausgabemodul mit einer Empfangseinheit für elektromagnetische Wellen, eine Tasche zur Aufbewahrung mindestens des Messgerätes und des Sprachausgabemoduls, mit einer ersten Halterung für das diagnostische Messgerät, die so ausgestaltet ist, dass das Gerät aus der Halterung herausnehmbar ist und bevorzugt nur in einer vorgegebenen Ausrichtung in der Halterung fixierbar ist, einer zweiten Halterung für das Sprachausgabemodul, die so ausgestaltet ist, dass das Modul herausnehmbar ist und bevorzugt nur in einer vorgegebenen Ausrichtung in der Halterung fixierbar ist. Dieses modulare System ist dadurch gekennzeichnet, dass die erste Halterung und die zweite Halterung so innerhalb der Tasche zueinander angeordnet sind, dass bei Einlegen der Geräte in die erste und die zweite Halterung ein drahtloser Datenaustausch durch die räumliche Ausrichtung der Sendeeinheit des diagnostischen Messgerätes und der Empfangseinheit des Sprachausgabemoduls zwischen den beiden Geräten stattfinden kann.

Dabei können das diagnostische Messgerät wie auch das Sprachausgabemodul sowohl mit einer Sender- als auch einer Empfangseinheit ausgerüstet sein, so dass ein Datenaustausch sowohl vom diagnostischen Messgerät zum Sprachausgabemodul stattfinden kann, als auch vom Sprachausgabemodul zum diagnostischen Messgerät. Die Möglichkeit zum Datenaustausch über eine Sende- bzw. Empfangseinheit sowohl im diagnostischen Messgerät wie auch in dem Sprachausgabemodul dient dazu, den Benutzer eine Möglichkeit zu geben, die Geräte innerhalb des Vorratsbehältnisses in Form einer Tasche zu benutzen.

Außerdem können durch die drahtlose Verbindung der Geräte Daten zwischen den Geräten ausgetauscht werden, egal ob die Tasche im offenen oder im geschlossenen Zustand vorliegt. Das diagnostische Messgerät kann dabei ein Blutglukosemessgerät sein, aber auch andere diagnostische Messgeräte sind möglich(beispielsweise Koagulationsmessgeräte oder Urinmessgeräte).

Die drahtlose Übertragung der Daten zwischen den Geräten kann durch jede Form von elektromagnetischen Wellen stattfinden, wie beispielsweise Infrarotstrahlung. Aber auch andere elektromagnetische Wellen, wie beispielsweise Funkwellen oder Ultraschall können zur Übertragung von Daten genutzt werden. Alternativ kann die Datenübertragung auch über Bluetooth Kommunikation stattfinden.

Bei der Verwendung von elektromagnetischen Strahlen ist zu beachten, dass die Strahlungsquellen (hier die Sendeeinheit) sowie die Strahlungsempfänger (hier die Empfangseinheit) einen Strahlungs- bzw. Empfangsbereich besitzen, der sowohl in seiner Reichweite als auch in der seitlichen. Ausdehnung begrenzt ist. Solche Strahlungs- quellen bzw. -empfänger besitzen einen Strahlungskegel, wie er dem Fachmann für unterschiedlichste Strahlungsquellen bekannt ist. Das bedeutet, dass die Strahlung nicht nur in genau eine Richtung aus der Strahlungsquelle ausgesendet wird sondern, dass auch Strahlung in einem von 0° abweichenden Winkel aus der Strahlungsquelle austritt. Das gleiche gilt für den Empfänger. Die Reichweite und der Winkel sind abhängig von verschiedenen Parametern der Geräte, wie beispielsweise der Intensität der Strahlungsquelle, der Positionierung des Senders bzw. Empfängers im Gerät sowie der Wellenlänge der elektromagnetischen Wellen. Die Intensität kann entweder durch die Wahl der Strahlungsquelle selbst geregelt werden oder durch Filter. Zur Begrenzung des Sende- bzw. Empfangswinkels können zusätzlich Blenden eingesetzt werden.

In einer bevorzugten Ausführungsform, in der die Reichweite der Sendeeinheit begrenzt werden soll, um nicht mit anderen Geräten in störenden Kontakt zu treten, wird bevorzugt Infrarotstrahlung zur Informationsübertragung eingesetzt. Bei Verwendung von Infrarotstrahlung ist die Ausrichtung der Sende- und Empfangseinheiten nötig. Aus diesem Grund ist in einer bevorzugten Ausführungsform die Sendeeinheit des Messgerätes und die Empfangseinheit des Sprachausgabemoduls so in den Geräten eingebracht, dass die Sende- bzw. Empfangseinheit beim Einbringen der Geräte in die Halterungen des modularen Systems, zueinander ausgerichtet sind und dass sich Sende- bzw. Empfangsbereich überlappen.

Durch diese Ausrichtung der beiden Geräte sind verschiedene Anordnungen der Halterungen in der Tasche möglich, je nachdem wo sich die Sende- bzw. Empfangseinheit in den beiden Geräten befinden. Bevorzugter weise sind dabei die Halterungen so ausgestaltet, dass die Geräte nur in einer Ausrichtung in der Halterung eingeführt und gehaltert werden. Dies ist besonders wichtig für Benutzer, die eine eingeschränkte Sehfähigkeit haben, da hier keine optische Kontrolle darüber stattfinden kann, ob sich die Geräte in der richtigen Halterung befinden. Wenn sich die Dimensionen der beiden Geräte ähneln, ist eine Ausgestaltung der Halterungen bevorzugter Weise so zu wählen, dass ein Vertauschen der beiden Geräte in der Tasche verhindert wird.

In einer bevorzugten Ausführungsform sind trotz dieser Ausrichtung der Geräte zueinander, die Funktionalitäten der Geräte weiterhin vom Benutzer auch im bevorrateten Zustand in der Tasche nutzbar. In einer besonders bevorzugten Ausführungsform sind die beiden Geräte und damit vorzugsweise auch die beiden Halterungen für die Geräte in einem Winkel zwischen 70° und 120°, bevorzugter Weise in einem Winkel von ca. 90° zueinander angeordnet. Durch diese Anordnung kann zum einen gewährleistet werden, dass alle Funktionalitäten der Geräte im bevorrateten Zustand nutzbar sind, hierdurch aber auch eine Platz sparende Anordnung gewährleistet ist.

In einer bevorzugten Ausführungsform besitzen sowohl das Sprachausgabemodul als auch das Messgerät jeweils eine Sende- und eine Empfangseinheit. Hierdurch kann ein Datenaustausch sowohl in die Richtung vom Messgerät zum Sprachausgabemodul als auch vom Sprachausgabemodul zum Messgerät gewährleistet werden. Dies ist besonders bevorzugt, wenn neben dem Datenaustausch vom Messgerät zum Sprachausgabemodul ebenfalls eine Kontrolle von übereinstimmenden Daten wie Uhrzeit und Datum gewährleistet werden soll, beziehungsweise eine Übertragung von gespeicherten Daten aus dem Sprachausgabemodul zum Messgerät und ebenfalls vom Sprachausgabemodul zum Messgerät möglich sein soll.

Durch die Ausrichtung der Geräte in einem vorbestimmten Winkel zueinander, ist es möglich, den Datenaustausch zwischen der Sendereinheit und der Empfangseinheit über eine räumlich ausgerichtete elektromagnetische Welle stattfinden zu lassen. Diese Ausrichtung der ausgestrahlten Wellen dient dazu, dass keine Geräte, die in unmittelbarer Nähe zu den benutzten Geräten liegen, störend beeinflusst werden oder von diesen gestört werden. Bevorzugter Weise sollte die Reichweite der Sendereinheiten nicht über das System hinausgehen. In einer bevorzugten Ausführungsform ist die Reichweite der Sendeeinheit nicht größer als einen halben Meter, in einer besonders bevorzugten Ausführungsform ist die Reichweite der Sendereinheit nicht größer als 10 cm. Wie bereits erwähnt, ist durch diese Minimierung der Reichweite der Sendeeinheit gewährleistet, dass ein Datenaustausch mit Geräten die in unmittelbarer Nähe zum System liegen, nicht stattfinden kann.

Um den ungewollten Datenaustausch mit anderen Sendegeräten weiterhin auszuschließen, kann das System weiterhin durch den Austausch eines Signals in Form einer Checksumme und/oder Kennung zwischen Messgerät und Sprachausgabemodul geschützt werden. Hierbei tauschen die Geräte beim Sendevorgang beispielsweise eine Codierung aus, die von dem empfangenden Gerät überprüft wird und hierdurch eine weitere Benutzung freigegeben wird.

### Sprachausgabemodul

Das Sprachausgabemodul dient dem Patienten sowohl zur Speicherung von Messdaten als auch zur Datenverarbeitung sowie der akustischen Ansage von Messdaten. Aufgrund der Datenverarbeitung, die in dem Sprachausgabemodul integriert sein kann, können zusätzliche Aufbereitungen von Messwerten die vom diagnostischen Messgerät übertragen wurden, vorgenommen werden. Die Größe des Sprachausgabemoduls ist bevorzugter weise so gewählt, dass ein Patient dieses Sprachausgabemodul leicht in einer Hand halten kann und dabei mit der anderen Hand Funktionstasten auf dem Sprachausgabemodul bedienen kann. Solch ein Sprachausgabemodul ist beispielsweise bekannt durch die US Patentanmeldung 2006/0277048. In dem Sprachausgabemodul kann darüber hinaus eine Speichereinheit zur Speicherung von Messdaten und anderen Daten vorhanden sein.

Weiterhin kann das Sprachausgabemodul eine Suchfunktion für gespeicherte Daten aufweisen, die es dem Patienten ermöglicht auf einfache Weise nach gespeicherten Daten zu suchen und diese anschließend akustisch ansagen zu lassen. Dabei kann das Sprachausgabemodul eine Menüfunktion besitzen, die es auch einem blinden oder sehbehinderten Benutzer ermöglicht leicht durch die Suchfunktion oder händisch mit den gespeicherten Daten umzugehen.

### Tasche

Die Tasche ist ein Bevorratungsbehältnis für die Aufbewahrung mindestens des Messgerätes und des Sprachausgabemoduls. Sie kann jede erdenkliche Form sowohl in Höhe wie in Breite als auch in Tiefe annehmen. Beispielsweise kann die Tasche in Form eines Quadrates, Rechteckes oder Kreisesausgestaltet sein. Bevorzugter weise besitzt die Tasche ein Bodenelement und einen Deckel, wobei bevorzugter weise das Bodenelement und der Deckel mindestens an einer Stelle fest miteinander verbunden sind. In einer bevorzugten Ausführungsform sind das Bodenelement und der Deckel darüber hinaus mit einem Schließmechanismus wie beispielsweise einem Reißverschluss, einem Klettverschluss oder einem Clipverschluss verschließbar miteinander verbunden, sodass die Tasche verschließbar und leicht zu reinigen ist, da es zu einer Verschmutzung mit Blut und anderen Substanzen kommen kann.

In dieser Tasche befinden sich mindestens 2 Halterungen zur lösbaren Fixierung des Messgerätes sowie des Sprachausgabemoduls. Diese Halterungen können sich entweder beide auf dem Bodenelement oder an dem Deckel der Tasche befinden Alternativ kann eine Halterung auf dem Bodenelement und die andere Halterung an dem Deckel befinden. Diese Halterungen sind so ausgestaltet, dass sie die Geräte auch bei leichten Erschütterungen in der Halterung fixieren können, die aber durch Aufwenden einer geringen Kraft, wie sie beispielsweise durch den Patienten ohne weiteres Werkzeug aufzubringen ist, wieder lösbar ist.

Die Halterungen können aus verschiedenen Materialien wie Stoffbändern, elastischen Gummibändern, Haken, Clips oder an die Form des Gerätes angepasste Plastikhalterungen sein. Außerdem können sich weitere Halterungen für zusätzliche Utensilien in der Tasche befinden wie z.B. Halterungen für Magazine zum Aufbewahren von Teststreifen oder Lanzetten sowie sonstigen Utensilien, die zur Benutzung für den Patienten wichtig sein können. Die beiden Halterungen für die Geräte sind so zueinander angeordnet, dass bei Einlegen der Geräte in die erste und die zweite Halterung ein drahtloser Datenaustausch durch die räumliche Ausrichtung des Senders der Sendeeinheit und des Empfängers der Empfangseinheit zwischen den beiden Geräten stattfinden kann. Diese Anordnung der Halterungen ist dabei dadurch charakterisiert, dass ein ungestörter Datenaustausch zwischen der Sendereinheit des einen Gerätes und der Empfangseinheit des anderen Gerätes stattfinden kann.

Des Weiteren sollen die Bedienelemente des Gerätes und der Empfangseinheit leicht zu bedienen sein. Je nachdem wo die Sende- bzw. Empfangseinheiten in den Geräten angebracht sind, können die Halterungen nebeneinander in der Tasche angeordnet sein oder hintereinander oder seitlich versetzt. Da sich in einer bevorzugten Ausführungsform die Sendereinheit des Messgerätes an einer der kurzen Querseiten des Gerätes befindet, und die Empfangseinheit des Sprachausgabemoduls sich an einem Ende der Längsseite befindet, sind die Halterungen in der Tasche so zueinander positioniert, dass die beiden Geräte in einem Winkel zwischen 70° und 120° besonders bevorzugt in einem Winkel von ca. 90° zueinander angeordnet sind, so dass Sender- und Empfangseinheiten der beiden Geräte aufeinander zugerichtet sind. Dabei sind die Halterungen so ausgestaltet, dass sie die Empfangs- bzw. Sendereinheit der Geräte nicht überdecken und außerdem die weiteren Funktionen der Messgeräte weiterhin zugänglich machen wie beispielsweise die Tastatur oder auch das Mikrofon bzw. das Display. Da sich die Empfangs- und Sendeeinheiten jedoch auch an anderer Stelle sowohl im Messgerät als auch im Sprachausgabemodul befinden können, ist auch eine seitliche Anordnung der Halterungen oder ein Hintereinanderliegen der Halterungen denkbar. Außerdem könnten die Sende- bzw. Empfangseinheiten sowohl über eine Seitenfläche als auch über eine Front- oder Rückseite des Messgerätes sich erstrecken, so dass auch einer Anordnung der Halterungen sowohl auf dem Bodenelement wie auf dem Deckel der Tasche denkbar sind, so dass sowohl ein Datenaustausch bei offener als auch bei geschlossener Tasche möglich ist.

Die Halterungen sind dabei bevorzugter weise so ausgestaltet, dass immer nur ein Gerät in jeweils eine Halterung eingebracht werden kann. Außerdem ist in einer bevorzugten Ausführungsform die Ausrichtung der Geräte durch die Form der Halterung vorgegeben. Durch diese eindeutige Ausgestaltung der Halterung ist ein falsches Einlegen der Geräte ausgeschlossen, was besonders für sehbehinderte Benutzer sehr von Vorteil ist, da sie nur umständlich überprüfen können, ob die Geräte richtig herum eingelegt sind und miteinander kommunizieren können.

Das Material der Tasche kann sowohl Kunststoff als auch Leder sein sowie Mischungen aus verschiedenen Materialien und soll leicht zu reinigen sein.

Das Bodenelement ist dabei in einer bevorzugten Ausführungsform so in die Tasche eingearbeitet, dass sie austauschbar ist und somit andere Bodenelemente mit beispielsweise unterschiedlich geformten bzw. ausgerichteten Halterungen verwendet werden können.

### Diagnostisches Messgerät

Das diagnostische Messgerät kann, wie bereits erwähnt, ein Messgerät für verschiedene Parameter für unterschiedliche Probematerialien sein. Das Probematerial kann beispielsweise aus verschiedenen Körperflüssigkeiten, wie Blut, Serum, Plasma oder auch Urin oder Speichelflüssigkeit bestehen. Das diagnostische Messgerät kann dabei sowohl zur Vermessung von einem oder auch mehreren Analyten benutzt werden. In einer bevorzugten Ausführungsform ist das diagnostische Messgerät ein Blutglukosemessgerät. Dies wird vor allem von Diabetikern zur Selbstkontrolle von Blutzuckermesswerten benutzt. Diese Geräte sind im Stand der Technik hinlänglich bekannt wie beispielsweise AccuChek Aviva, AccuChek Compact oder andere im Handel erhältlichen Glucosemessgeräte für Diabetiker.

### Figurenbeschreibung

### Bezugszeichen

| | | | |
|---|---|---|---|
| 1 | Tasche | 7b | zweite Halterung Sprachausgabemodul |
| 2 | Messgerät | 7c | Halterung Utensilien |
| 2a | Kopfende | 8 | Deckel |
| 2b | Fußende | 9 | Bodenelement |
| 3 | Sprachausgabemodul | 10 | Netz |
| 4 | Vorratsbehältnis | 11 | Tragegriff |
| 5 | Reißverschluss | 30 | Schnittstelle Sende-/Empfangseinheit Messgerät |
| 6 | Stechhilfe | | |
| 7a | erste Halterung Messgerät | 31 | Schnittstelle Sende-/Empfangseinheit Sprachausgabemodul |

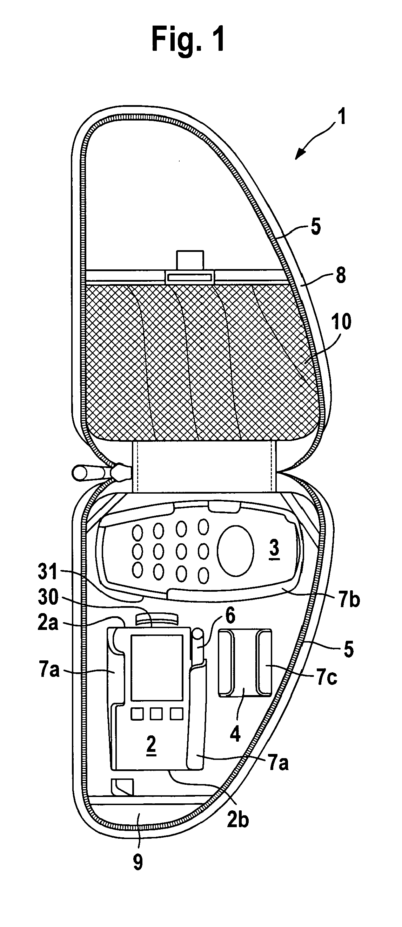

In Figur 1 ist ein Beispiel eines modularen Systems abgebildet, das eine Tasche (1) sowie ein Messgerät (2) mit einer Schnittstelle (30), ein Sprachausgabemodul (3) mit einer Schnittstelle (31) sowie ein Vorratsbehältnis (4) für Testelemente beinhaltet. Die Schnittstellen (30, 31) können dabei entweder Sende- oder Empfangseinheiten bzw. kombinierte Sende/Empfangseinheiten sein. Die Geräte sowie das Vorratsbehältnis (4) sind in Halterungen (7a, 7b, 7c) in der Tasche (1) fixiert. Die Tasche (1) weist einen Deckel (8) sowie ein Bodenelement (9) auf, die miteinander verbunden sind und zum Verschließen der Tasche (1) aufeinander gelegt werden können. Zum Verschließen der Tasche (1) befindet sich ein Reißverschluss (5) zwischen dem Deckel (8) und dem Bodenelement (9) der Tasche (1). Neben einem Reißverschluss (5) kann alternativ ein Druckknopf, ein Klettverschluss oder ein Clip verwendet werden, der die beiden Teile der Tasche (1) in ihrer Position zueinander fixiert und die Tasche verschließt. In Figur 1 sind die beiden Geräte, das Messgerät (2) sowie das Sprachausgabemodul (3) in einem Winkel von ca. 90° zueinander angeordnet. Dies ist eine bevorzugte Ausführungsform, da sich am Messgerät (2) eine Infrarotschnittstelle (30) am Kopf, hier Kopfseite (2a) genannt, des Gerätes befindet, während sich beim Sprachausgabemodul (3) diese Schnittstelle (31) an der Seite des Gerätes (3) befindet. Die Anordnung der Infrarotschnittstelle (30) an der Kopfseite (2a) des Messgerätes (2) ist dadurch begründet, dass seitlich zu dem Messgerät (2) eine Stechhilfe (6) angeordnet ist und an dem Kopfende (2a) gegenüberliegenden Fußende (2b) des Messgerätes (2) die Öffnung zur Einführung eines Testelementes angebracht ist. Die Anordnung der beiden Geräte in einem 90 Grad Winkel ist besonders Platz sparend und ermöglicht die Verwendung sowohl des Sprachausgabemoduls (3) als auch das Messgerätes (2) im bevorrateten Zustand. So ist es dem Benutzer möglich, die Blutgewinnung über die Stechhilfe (6) sowohl von dem Fußende (2b) aus zu realisieren, sowie das Spannen und Auslösen der Stechhilfe (6) von der Kopfseite (2a) durchzuführen. Diese Anordnung der Geräte ist jedoch nicht einschränkend zu sehen, denn es wäre ebenso möglich, die Schnittstellen (30, 31) an den Geräten (2, 3) an anderer Stelle anzubringen und demzufolge die Halterungen (7a, 7b) in der Tasche (1) so zu variieren, dass eine störfreie Kommunikation zwischen den beiden Geräten (2, 3) weiterhin möglich ist. Die Halterungen (7a, 7b) sind dabei so ausgestaltet und angeordnet, dass sie das Bedienen der Geräte (2, 3) sowie der Vorratsbehältnisse (4) im bevorrateten Zustand ermöglichen und dabei eine störfreie Kommunikation zwischen den Geräten gewährleisten. In dem hier gezeigten Beispiel sind die Halterungen (7a, 7b, 7c) aus Plastik ausgestaltet, dass so gefertigt ist, dass die Geräte (2, 3) sowie das Vorratsbehältnis (4) leicht aus den Halterungen lösbar sind. Halterung durch beispielsweise Gummibänder oder Klettbänder oder sonstigen Elementen, die eine ausreichende Fixierung mit einfacher Lösbarkeit verbinden, sind ebenfalls denkbar. Die Halterungen (7a, 7b) sind bevorzugter weise dabei so ausgestaltet, dass nur eines der Geräte fixiert werden kann und dass das Gerät auch nur in einer Richtung in der Halterung, beispielsweise das Messgerät (2) in die Halterung (7a), positioniert werden kann, sodass gewährleistet ist, dass die Schnittstellen (30, 31) aufeinander zu gerichtet sind. Durch die Anordnung der Geräte in einem 90° Winkel zueinander, ist selbst einem blinden Benutzer die direkte Zuordnung von Sprachausgabemodulen (3) und Messgerät (2) möglich. Neben den hier gezeigten Geräten (2, 3) und Utensilien (4) die in der Tasche (1) fixiert und bevorratet werden können, können noch weitere Gegenstände mitgeführt werden. Dies ist beispielsweise möglich, in dem ein Netz (10) im Deckel (8) der Tasche in (1) angebracht ist. Es ist weiterhin denkbar, dass weitere Halterungen für zusätzliche Elemente in den Deckel (8) oder das Bodenelement (9) in die Tasche (1) eingebracht werden können.

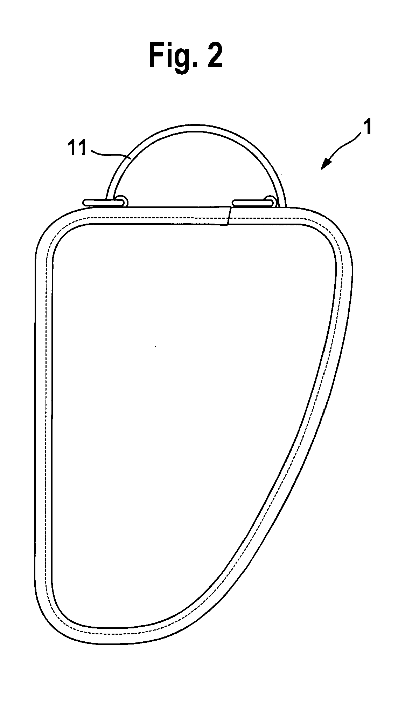

In Figur 2 ist die Tasche (1) im geschlossenen Zustand dargestellt, wobei sich ein Tragegriff (11) am Deckel (8) oder Bodenelement (9) der Tasche (1) befindet. Dieser Tragegriff (11) kann in Form eines Bandes ausgestaltet sein oder in Form eines festen Griffes oder sonstiger Halterungen, die das Tragen der Tasche (1) erleichtern.

## Patentansprüche

1. Modulares System zur akustischen Ausgabe von diagnostischen Messwerten, enthaltend:
- ein diagnostisches Messgerät (2) mit einer Sendeeinheit für elektromagnetische Wellen (30),
- ein Sprachausgabemodul (3) mit einer Empfangseinheit für elektromagnetische Wellen (31),
- eine Tasche (1) zur Aufbewahrung mindestens des Messgerätes (2) und des Sprachausgabemoduls (3), mit
- einer ersten Halterung (7a) für das diagnostische Messgerät, die so ausgestaltet ist, dass das Gerät aus der Halterung herausnehmbar ist und in einer vorgegebenen Ausrichtung in der Halterung fixiert wird,
- einer zweiten Halterung (7b) für das Sprachausgabemodul, die so ausgestaltet ist, dass das Gerät herausnehmbar ist und nur in einer vorgegebenen Ausrichtung in der Halterung fixiert wird,
**dadurch gekennzeichnet, dass** die erste Halterung (7a) und die zweite Halterung (7b) so innerhalb der Tasche (1) zu einander angeordnet sind, dass bei Einlegen der Geräte in die erste und die zweite Halterung ein drahtloser Datenaustausch durch die räumliche Ausrichtung des Senders der Sendeeinheit und des Empfängers der Empfangseinheit zwischen den beiden Geräten stattfinden kann.

2. System nach Anspruch 1, **dadurch gekennzeichnet, dass** der Datenaustausch über Infrarot erfolgt.

3. System nach Anspruch 1 oder 2, **dadurch gekennzeichnet, dass** das diagnostische Messgerät (2) ein Blutglukosemessgerät ist.

4. System nach einem der vorherigen Ansprüche, **dadurch gekennzeichnet, dass** das Messgerät (2) zusätzlich eine Empfangseinheit (30) besitzt.

5. System nach einem der vorherigen Ansprüche, **dadurch gekennzeichnet, dass** das Sprachmodul (3) zusätzlich eine Sendeeinheit (31) und/oder eine Datenverarbeitungseinheit besitzt.

6. System nach einem der vorherigen Ansprüche, **dadurch gekennzeichnet, dass** der Datenaustausch zwischen einer der Sendeeinheiten (30) und einer der Empfangseinheiten (31) über eine räumlich ausgerichtete elektromagnetische Welle stattfindet.

7. System nach einem der vorherigen Ansprüche, **dadurch gekennzeichnet, dass** die Reichweite der Sendeeinheiten (30) nicht über die Ausdehnung des Systems hinausgeht.

8. System nach einem der vorherigen Ansprüche, **dadurch gekennzeichnet, dass** das Sprachausgabemodul (3) eine Speichereinheit aufweist.

9. System nach Anspruch 6, **dadurch gekennzeichnet, dass** das Sprachausgabemodul (3) eine Suchfunktion für gespeicherte Daten aufweist.

10. System nach einem der vorherigen Ansprüche, **dadurch gekennzeichnet, dass** in der Tasche (1) weitere Halterungen (7c) für zusätzliche Utensilien, wie beispielsweise Magazine für Teststreifen, enthalten sind.

11. System nach einem der vorherigen Ansprüche, **dadurch gekennzeichnet, dass** die Geräte beim Sendevorgang eine Codierung austauschen.

12. System nach Anspruch 6, **dadurch gekennzeichnet, dass** durch die Codierung ein Austausch mit anderen Sendegeräten ausgeschlossen ist.

13. System nach einem der vorherigen Ansprüche, **dadurch gekennzeichnet, dass** an dem Messgerät (2) ablösbar zusätzlich eine Stechhilfe (6) angebracht ist.

14. System nach einem der vorherigen Ansprüche, **dadurch gekennzeichnet, dass** das diagnostische Messgerät (2) und/oder die Stechhilfe (6) benutzt werden kann, ohne aus der Halterung (7a) genommen zu werden.

15. System nach einem der vorherigen Ansprüche, **dadurch gekennzeichnet, dass** die Tasche (1) ein Bodenelement (9) und ein Deckel (8) besitzt, die gegeneinander beweglich sind.

16. System nach einem der vorherigen Ansprüche, **dadurch gekennzeichnet, dass** die Tasche (1) verschließbar ist.

17. System nach einem der vorherigen Ansprüche, **dadurch gekennzeichnet, dass** die Tasche (1) durch einen Klipp oder einen Klettverschluss oder einen Reißverschluss (5) verschließbar ist.

18. System nach einem der vorherigen Ansprüche, **dadurch gekennzeichnet, dass** die Tasche (1) leicht zu reinigen ist.

19. System nach Anspruch 15, **dadurch gekennzeichnet, dass** das Bodenelement (9) austauschbar ist.

20. System nach einem der vorherigen Ansprüche, **dadurch gekennzeichnet, dass** die Halterungen (7a, 7b) so zueinander angeordnet sind, dass das Bedienen der Geräte (2, 3) im bevorrateten Zustand möglich ist.
